# EUROPEAN PATENT APPLICATION

(11) **EP 4 230 256 A1**
(43) Date of publication of application: **23.08.2023**
(21) Application number: 23156692.8
(22) Date of filing: 15.02.2023
(51) Int. Cl.: A61N 1/36, A61N 1/05, A61N 1/08, A61N 1/372

(54) **THERAPEUTIC ELECTRODE LOCATION PREDICTION VISUALIZATION USING MACHINE LEARNING**

(30) Priority: 21.02.2022 US 202263268300 P; 26.01.2023 US 202318101885
(71) Applicant: Medtronic, Inc, Minneapolis, MN 55432 (US)
(72) Inventor: Case, Michelle A., Minneapolis (US); Panken, Eric J., Minneapolis (US); Molina, Rene A., Minneapolis (US); Dassbach Green, Paula A.E., Minneapolis (US); Becker, Abbey Beuning Holt, Minneapolis (US)
(74) Representative: Zimmermann & Partner Patentanwälte mbB

(57) **Abstract**

Systems and methods for programming an implantable medical device comprising a simulated environment with at least one lead having a plurality of electrodes, computing hardware of at least one processor and a memory operably coupled to the at least one processor, and instructions that, when executed on the computing hardware, cause the computing hardware to implement a training sub-system configured to conduct a brain sense survey using the simulated environment, develop at least one machine learning model based on the brain sense survey, apply the at least one machine learning model to in-vivo patient data to determine at least one predicted electrode from the plurality of electrodes relative to an oscillatory source, visualize the at least one predicted electrode, and program a medical device based on the at least one predicted electrode.

## Description

### CROSS-REFERENCE TO RELATED APPLICATIONS

This application claims the benefit of U.S. Provisional Application Serial No. 63/268,300, filed February 21, 2022, the disclosure of which is hereby incorporated by reference in its entirety.

### FIELD

The present technology is generally related to implantable medical devices and, more particularly, implantable medical devices providing electrical stimulation therapy.

### BACKGROUND

Implantable medical devices, such as electrical stimulators or therapeutic agent delivery devices, have been proposed for use in different therapeutic applications, such as deep brain stimulation (DBS), spinal cord stimulation (SCS), pelvic stimulation, gastric stimulation, peripheral nerve stimulation, functional electrical stimulation or delivery of pharmaceutical agents, insulin, pain relieving agents or anti-inflammatory agents to a target tissue site within a patient. In some therapy systems, an implantable electrical stimulator delivers electrical therapy to a target tissue site within a patient with the aid of one or more electrodes, which may be deployed by medical leads and/or on a housing of the electrical stimulator, or both. In some therapy systems, therapy may be delivered via particular combinations of the electrodes carried by leads and/or by the housing of the electrical stimulator.

During a programming session, which may occur during implant of the medical device, during a trial session, or during an in-clinic or remote follow-up session after the medical device is implanted in the patient, a clinician may generate one or more therapy programs (also referred to as therapy parameter sets) that are found to provide efficacious therapy to the patient, where each therapy program may define values for a set of therapy parameters. A medical device may deliver therapy to a patient according to one or more stored therapy programs. In the case of electrical stimulation, the therapy parameters may define characteristics of the electrical stimulation waveform to be delivered. In examples in which electrical stimulation is delivered in the form of electrical pulses, for example, the therapy parameters may include an electrode configuration including an electrode combination and electrode polarities, an amplitude, which may be a current or voltage amplitude, a pulse width, and a pulse rate.

Optimizing therapy for DBS can be challenging and has become more challenging with the introduction of segmented leads. Additional contacts means that more time is needed for monopolar reviews, which is a technique used in the clinic to determine the optimal electrode for therapy. Directional stimulation also means more exploration and programming time is needed to evaluate that the therapy settings are effective and efficient. Selection of effective stimulation parameters for DBS therapy can therefore be time-consuming (e.g. longer and more frequent medical visits) for both the physician (also referred to as a clinician) and the patient.

Accordingly, there is a need to improve the efficiency of DBS programming.

### SUMMARY

In one aspect, the techniques of this disclosure solve the aforementioned needs of the industry. Embodiments utilize sensing data in a simulated environment to inform programming decisions by applying machine learning algorithms and visualizing the results for straightforward interpretation by a clinician.

In another aspect, machine learning can be used to detect the electrodes nearest (or furthest) from a physiological oscillatory source. In an embodiment, electrode rankings can be visualized with or without anatomical data such as imaging scan data.

In another aspect, machine learning can further inform the choices of stimulation parameters through visual targeting. For example, a physiological signal can be viewed easily within the physical context of anatomy (or, without anatomy).

In another aspect, machine learning can further automate the selection of the stimulation contact closest to the responsive tissues. In an embodiment, the location of sources can be visualized with or without anatomical imaging scan data. Various longitudinal changes associated with disease progression and therapy changes can be highlighted. Further, the changes in location of sources that may indicate changes in implant location, physiological changes, or other clinically relevant variables can be informed.

In one aspect, the present disclosure provides a method for programming a medical device comprising conducting a brain sense survey with at least one lead including a plurality of electrodes in a simulated environment, developing at least one machine learning model based on the brain sense survey, applying the at least one machine learning model to in-vivo patient data to determine at least one predicted electrode from the plurality of electrodes relative to an oscillatory source, visualizing the at least one predicted electrode, and programming the medical device based on the at least one predicted electrode.

In one aspect, the present disclosure provides a system, comprising a simulated environment with at least one lead having a plurality of electrodes, computing hardware of at least one processor and a memory operably coupled to the at least one processor, and instructions that, when executed on the computing hardware, cause the computing hardware to implement a training sub-system configured to conduct a brain sense survey using the simulated environment, develop at least one machine learning model based on the brain sense survey, apply the at least one machine learning model to in-vivo patient data to determine at least one predicted electrode from the plurality of electrodes relative to an oscillatory source, visualize the at least one predicted electrode, and program a medical device based on the at least one predicted electrode.

Accordingly, DBS programming burden can be reduced by automating the programming process and, further, by optimizing therapy settings.

Systems and methods for programming an implantable medical device comprising a simulated environment with at least one lead having a plurality of electrodes, computing hardware of at least one processor and a memory operably coupled to the at least one processor, and instructions that, when executed on the computing hardware, cause the computing hardware to implement a training sub-system configured to conduct a brain sense survey using the simulated environment, develop at least one machine learning model based on the brain sense survey, apply the at least one machine learning model to in-vivo patient data to determine at least one predicted electrode from the plurality of electrodes relative to an oscillatory source, visualize the at least one predicted electrode, and program a medical device based on the at least one predicted electrode. The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1A is a schematic illustrating an example deep brain stimulation (DBS) system configured to deliver electrical stimulation therapy to a tissue site within a brain of a patient, according to an embodiment.
FIG. 1B is a block diagram illustrating components of the system of FIG. 1A, according to an embodiment.
FIG. 2 is a block diagram of a system configured to deliver electrical stimulation therapy to a tissue site within a brain of a patient, according to an embodiment.
FIG. 3 is a block diagram of a training system for a system for delivering electrical stimulation therapy, according to an embodiment.
FIG. 4A is an annotated diagram of an electrode and the cardinal directions of the electrode, according to an embodiment.
FIG. 4B is a diagram of an example lead utilized in the training system of FIG. 3, according to an embodiment.
FIG. 4C is an annotated illustration of a cross-section of the lead of FIG. 4B, according to an embodiment.
FIG. 5 is a flowchart of a method for programming a medical device, according to an embodiment.
FIG. 6 is a flowchart of another method for programming a medical device, according to an embodiment.
FIGS. 7A-7C are example location prediction visualizations for a lead, according to embodiments.
FIGS. 8A-8B are example electrode prediction visualizations for a lead, according to embodiments.

While various embodiments are amenable to various modifications and alternative forms, specifics thereof have been shown by way of example in the drawings and will be described in detail. It should be understood, however, that the intention is not to limit the claimed inventions to the particular embodiments described. On the contrary, the intention is to cover all modifications, equivalents, and alternatives falling within the spirit and scope of the subject matter as defined by the claims.

### DETAILED DESCRIPTION

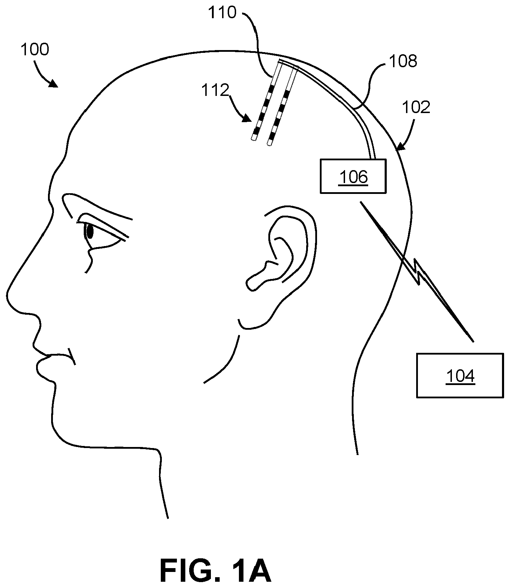

Referring to FIG. 1A, an example deep brain stimulation (DBS) system 100 configured to deliver electrical stimulation therapy to a tissue site within a brain of a patient 102 is depicted, according to an embodiment. Patient 102 ordinarily will be a human patient. In some cases, however, therapy system 100 can be applied to other mammalian or non-mammalian non-human patients. In the example shown in FIG. 1A, therapy system 100 includes medical device programmer 104, implantable medical device (IMD) 106, lead extension 108, and one or more leads 110 with respective sets of electrodes 112. IMD 106 includes a stimulation generator configured to generate and deliver electrical stimulation therapy to one or more regions of brain of patient 102 via one or more electrodes 112 of one or more leads 110, respectively.

DBS can be used to treat or manage various patient conditions, such as, but not limited to, seizure disorders (e.g., epilepsy), pain, migraine headaches, psychiatric disorders (e.g., major depressive disorder (MDD), bipolar disorder, anxiety disorders, post-traumatic stress disorder, dysthymic disorder, and obsessive compulsive disorder (OCD)), behavior disorders, mood disorders, memory disorders, mentation disorders, movement disorders (e.g., essential tremor or Parkinson's disease), Huntington's disease, Alzheimer's disease, or other neurological or psychiatric disorders and impairment of patient 102.

Therapy systems configured for treatment of other patient conditions via delivery of therapy to the brain or another suitable target therapy delivery site in patient 102 can also be used in accordance with the techniques disclosed herein. For example, in other applications of therapy system 100, the target therapy delivery site within patient 102 can be a location proximate to a spinal cord or to sacral nerves (e.g., the S2, S3 or S4 sacral nerves) in patient 102 or any other suitable nerve, organ, muscle or muscle group in patient 102, which can be selected based on, for example, a patient condition. For example, therapy system 100 can be used to deliver electrical stimulation or a therapeutic agent to tissue proximate to a pudendal nerve, a perineal nerve or other areas of the nervous system, in which cases, leads 110 are implanted and substantially fixed proximate to the respective nerve. As further examples, an electrical stimulation system may be positioned to deliver a stimulation to help manage peripheral neuropathy or post-operative pain mitigation, ilioinguinal nerve stimulation, intercostal nerve stimulation, gastric stimulation for the treatment of gastric mobility disorders and obesity, urinary dysfunction, fecal dysfunction, sexual dysfunction, muscle stimulation, for mitigation of other peripheral and localized pain (e.g., leg pain or back pain).

Leads 110 can be positioned to deliver electrical stimulation therapy to one or more target tissue sites within the brain to manage patient symptoms associated with a disorder of patient 102. Leads 110 may be implanted to position electrodes 112 at desired locations of the brain via any suitable technique, such as through respective burr holes in the skull of patient 102 or through a common burr hole in the cranium. Leads 110 can be placed at any location within the brain such that electrodes 112 are capable of providing electrical stimulation to target therapy delivery sites within the brain during treatment. Different neurological, motor, or psychiatric disorders can be associated with activity in one or more of regions of the brain, which may differ between patients. Accordingly, the target therapy delivery site for electrical stimulation therapy delivered by leads 110 may be selected based on the patient condition. For example, a suitable target therapy delivery site within the brain for controlling a movement disorder of patient 102 may include one or more of the pedunculopontine nucleus (PPN), thalamus, basal ganglia structures (e.g., globus pallidus, substantia nigra or subthalamic nucleus (STN)), zona inserta, fiber tracts, lenticular fasciculus (and branches thereof), ansa lenticularis, or the Field of Forel (thalamic fasciculus). The PPN may also be referred to as the pedunculopontine tegmental nucleus.

IMD 106 can deliver electrical stimulation therapy to the brain of patient 102 according to one or more stimulation therapy programs (also referred to herein as "set of stimulation parameter values"). A stimulation therapy program may define one or more electrical stimulation parameter values for therapy generated by a stimulation generator of IMD 106 and delivered from IMD 106 to a target therapy delivery site within patient 102 via one or more electrodes 112. The electrical stimulation parameters may define an aspect of the electrical stimulation therapy, and may include, for example, voltage or current amplitude of an electrical stimulation signal, a charge level of an electrical stimulation, a frequency of the electrical stimulation signal, waveform shape, on/off cycling state (e.g., if cycling is "off," stimulation is always on, and if cycling is "on," stimulation is cycled on and off) and, in the case of electrical stimulation pulses, pulse rate, pulse width, and other appropriate parameters such as duration or duty cycle. In addition, if different electrodes are available for delivery of stimulation, a therapy parameter of a therapy program can be further characterized by an electrode combination, which can define selected electrodes 112 and their respective polarities. In some examples, stimulation may be delivered using a continuous waveform and the stimulation parameters can define this waveform.

In addition to being configured to deliver therapy to manage a disorder of patient 102, therapy system 100 can be configured to sense bioelectrical brain signals or another physiological parameter of patient 102. For example, IMD 106 can include a sensing engine that is configured to sense bioelectrical brain signals within one or more regions of the brain via electrodes 112. Accordingly, in some examples, electrodes 112 can be used to deliver electrical stimulation to target sites within the brain as well as sense brain signals. However, IMD 106 can also use a separate set of sensing electrodes to sense the bioelectrical brain signals. In some examples, the sensing engine of IMD 106 can sense bioelectrical brain signals via one or more of the electrodes 112 that are also used to deliver electrical stimulation to the brain. In other examples, one or more of electrodes 112 can be used to sense bioelectrical brain signals while one or more different electrodes 112 can be used to deliver electrical stimulation.

External medical device programmer 104 is configured to wirelessly communicate with IMD 106 as needed to provide or retrieve therapy information. Programmer 104 is an external computing device that the user, e.g., the clinician and/or patient 102, can use to communicate with IMD 106. For example, programmer 104 can be a clinician programmer that the clinician uses to communicate with IMD 106 and program one or more therapy programs for IMD 106. In addition, or instead, programmer 104 can be a patient programmer that allows patient 102 to select programs and/or view and modify therapy parameter values. The clinician programmer can include more programming features than the patient programmer. In other words, more complex or sensitive tasks may only be allowed by the clinician programmer to prevent an untrained patient from making undesired changes to IMD 106.

Programmer 104 can be a hand-held computing device with a display viewable by the user and an interface for providing input to programmer 14 (i.e., a user input mechanism). For example, programmer 104 can include a small display screen (e.g., a liquid crystal display (LCD) or a light emitting diode (LED) display) that presents information to the user. In addition, programmer 104 can include a touch screen display, keypad, buttons, a peripheral pointing device, voice activation, or another input mechanism that allows the user to navigate through the user interface of programmer 104 and provide input. If programmer 104 includes buttons and a keypad, the buttons may be dedicated to performing a certain function, e.g., a power button, the buttons and the keypad may be soft keys that change in function depending upon the section of the user interface currently viewed by the user, or any combination thereof.

In other examples, programmer 104 can be a larger workstation or a separate application within another multi-function device, rather than a dedicated computing device. For example, the multi-function device may be a notebook computer, tablet computer, workstation, one or more servers, cellular phone, personal digital assistant, or another computing device that may run an application that enables the computing device to operate as a secure medical device programmer 104. A wireless adapter coupled to the computing device can enable secure communication between the computing device and IMD 106.

When programmer 104 is configured for use by the clinician, programmer 104 may be used to transmit programming information to IMD 106. Programming information can include, for example, hardware information, such as the type of leads 110, the arrangement of electrodes 112 on leads 110, the position of leads 110 within the brain, one or more therapy programs defining therapy parameter values, therapeutic windows for one or more electrodes 112, and any other information that may be useful for programming into IMD 106. Programmer 104 can also be capable of completing functional tests (e.g., measuring the impedance of electrodes 112 of leads 110).

The clinician can also generate and store therapy programs within IMD 106 with the aid of programmer 104. Programmer 104 can assist the clinician in the creation/identification of therapy programs by providing a system for identifying potentially beneficial therapy parameter values. For example, during a programming session, programmer 104 may automatically select a combination of electrodes for delivery to therapy to the patient.

Programmer 104 can also be configured for use by patient 102. When configured as a patient programmer, programmer 104 can have limited functionality (compared to a clinician programmer) in order to prevent patient 102 from altering critical functions of IMD 106 or applications that may be detrimental to patient 102.

Whether programmer 104 is configured for clinician or patient use, programmer 104 is configured to communicate with IMD 106 and, optionally, another computing device, via wireless communication. Programmer 104, for example, may communicate via wireless communication with IMD 106 using radio frequency (RF) and/or inductive telemetry techniques known in the art, which can comprise techniques for proximal, mid-range, or longer-range communication. Programmer 104 can also communicate with another programmer or computing device via a wired or wireless connection using any of a variety of local wireless communication techniques, such as RF communication according to 802.11 or Bluetooth specification sets, infrared (IR) communication, or other standard or proprietary telemetry protocols. Programmer 104 can also communicate with other programming or computing devices via exchange of removable media, such as magnetic or optical disks, memory cards, or memory sticks. Further, programmer 104 can communicate with IMD 106 and another programmer via remote telemetry techniques known in the art, communicating via a personal area network (PAN), a local area network (LAN), wide area network (WAN), public switched telephone network (PSTN), or cellular telephone network, for example.

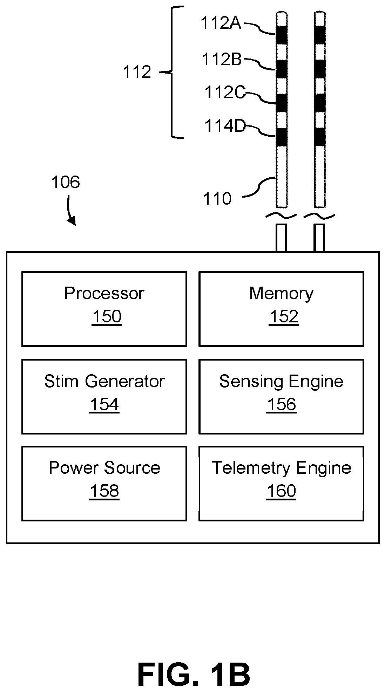

Referring to FIG. 1B, a block diagram illustrating components of system 100 of FIG. 1A is depicted, according to an embodiment. In an embodiment, IMD 106 generally includes a processor 150, memory 152, a stimulation generator 154, a sensing engine 156, a power source 158, and a telemetry engine 160.

Processor 150 can include one or more digital signal processors (DSPs), general purpose microprocessors, application specific integrated circuits (ASICs), field programmable logic arrays (FPGAs), or other equivalent integrated or discrete logic circuitry, or combinations thereof. The functions attributed to processors described herein may be provided by a hardware device and embodied as software, firmware, hardware, or any combination thereof. Processor 150 is configured to control stimulation generator 154 according to therapy programs stored by memory 152 to apply particular stimulation parameter values specified by one or more programs, such as amplitude, pulse width, and pulse rate.

Memory 152 can be operably coupled to processor 150 and can include any volatile or non-volatile media, such as a random access memory (RAM), read only memory (ROM), non-volatile RAM (NVRAM), electrically erasable programmable ROM (EEPROM), flash memory, and the like. Memory 152 can store computer-readable instructions that, when executed by processor 150, cause IMD 106 to perform various functions described herein.

In an embodiment, memory 152 can store therapy programs, operating instructions, and the like. Each stored therapy program defines a particular program of therapy in terms of respective values for electrical stimulation parameters, such as an electrode combination, current or voltage amplitude, and, if stimulation generator 154 generates and delivers stimulation pulses, the therapy programs can define values for a pulse width, and pulse rate of a stimulation signal. Each stored therapy program can also be referred to as a set of stimulation parameter values. Operating instructions guide general operation of IMD 106 under control of processor 150, and can include instructions for monitoring brain signals within one or more brain regions via electrodes 112 and delivering electrical stimulation therapy to patient 102

Stimulation generator 154, under the control of processor 150, is configured to generate stimulation signals for delivery to patient 102 via selected combinations of electrodes 112.

Sensing engine 156, under the control of processor 150, is configured to sense bioelectrical brain signals of patient 102 via electrodes 112.

Power source 158 delivers operating power to various components of IMD 106. Power source 158 can include a small rechargeable or non-rechargeable battery and a power generation circuit to produce the operating power. Recharging can be accomplished through proximal inductive interaction between an external charger and an inductive charging coil within IMD 106. In some examples, power requirements may be small enough to allow IMD 106 to utilize patient motion and implement a kinetic energy-scavenging device to trickle charge a rechargeable battery. In other examples, traditional batteries may be used for a limited period of time.

Telemetry engine 160 is configured to support wireless communication between IMD 106 and an external programmer 104 or another computing device under the control of processor 150. Processor 150 can receive, as updates to programs, values for various stimulation parameters such as amplitude and electrode combination, from programmer 104 via telemetry engine 160.

As depicted, system 100 can further comprise electrodes 112 of lead 110 that includes electrodes 112A-112D. Processor 150 can apply the stimulation signals generated by stimulation generator 154 to a selected combination of electrodes 112A-112D.

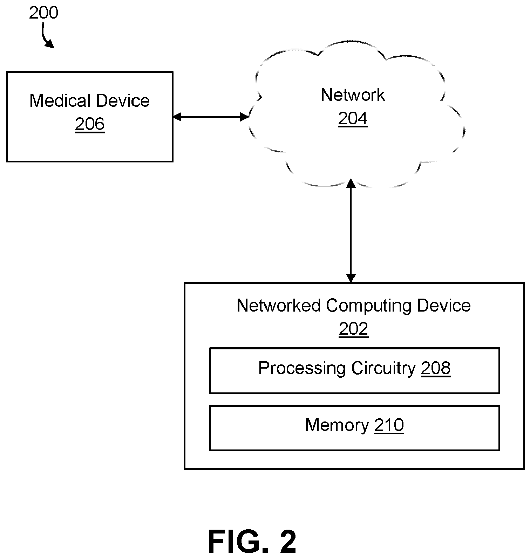

Referring to FIG. 2, a block diagram of a system 200 configured to deliver electrical stimulation therapy to a tissue site within a brain of a patient is depicted, according to an embodiment. In an embodiment, system 200 can include a networked computing device 202, a network 204, and a medical device 206.

Networked computing device 202 generally comprises processing circuitry 208 and memory 210. Of course, one of skill in the art will appreciate that networked computing device 202 can further comprise communication circuitry, a user interface, and a power source (not shown).

Processing circuitry 208 can include one or more processors that are configured to implement functionality and/or process instructions for execution within networked computing device 202. For example, processing circuitry 208 can be capable of processing instructions stored in memory 210. Processing circuitry 208 can include, for example, microprocessors, DSPs, ASICs, FPGAs, or equivalent discrete or integrated logic circuitry, or a combination of any of the foregoing devices or circuitry. Accordingly, processing circuitry 208 can include any suitable structure, whether in hardware, software, firmware, or any combination thereof, to perform the functions ascribed herein to processing circuitry 208.

Memory 210 can be configured to store information within networked computing device 202 during operation. Memory 210 can include a computer-readable storage medium or computer-readable storage device. In some examples, memory 210 includes one or more of a short-term memory or a long-term memory. Memory 210 can include, for example, RAM, dynamic random access memories (DRAM), static random access memories (SRAM), magnetic discs, optical discs, flash memories, or forms of electrically programmable memories (EPROM) or EEPROM. In some examples, memory 210 is used to store data indicative of instructions for execution by processing circuitry 208. Memory 210 can be used by software or applications running on networked computing device 202 to temporarily store information during program execution.

Network 204 comprises a communication network for connecting networked computing device 202 with medical device 206 (e.g. a wireless communication network, a wired communication network, a cellular communication network, the Internet, a short-range radio network (e.g., via Bluetooth)).

With reference to networked computing device 202 and network 204, embodiments of and the corresponding methods of configuring and operating system 200 can be performed in cloud computing, client-server, or other networked environment, or any combination thereof. The components of the system can be located in a singular "cloud" or network, or spread among many clouds or networks. End-user knowledge of the physical location and configuration of components of the system is not required.

For ease of explanation, medical device 206 is labeled separately from previously-described IMD 106, but one of ordinary skill in the art will readily understand that medical device 206 can be substantially similar to IMD 106 as depicted and described in FIGS. 1A-1B.

In an embodiment, networked computing device 202 can be configured to coordinate bench top simulations to create machine learning algorithms to predict electrode location of medical device 206 in-vivo. In certain embodiments, bench top data is relatively easy to acquire and can be used to optimize machine learning algorithms and/or machine learning models, which can subsequently by applied to in-vivo models in medical device 206. Accordingly, networked computing device 202 can further implement or be coupled to a training data system or subsystem, as will be described further herein.

As used herein, the term "machine learning algorithm" can include procedures that are implemented in code and are run on data. A "machine learning model" is output by machine learning algorithms and are comprised of model data and a prediction algorithm.

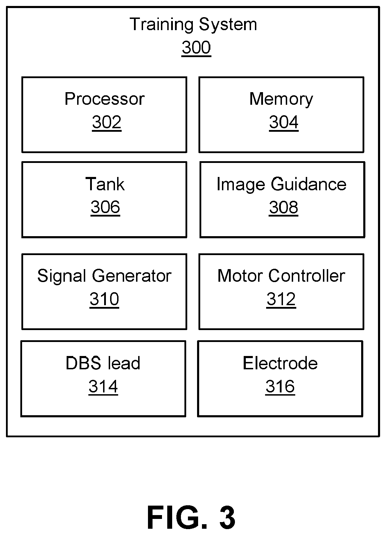

Referring to FIG. 3, a block diagram of a training system 300 is depicted, according to an embodiment. In an embodiment, training system 300 can generally comprise or can effectively be networked computing device 202. Accordingly, training system 300 comprises processor 302 and memory 304 as described with respect to networked computing device 202. Training system 300 can further comprise input/output components (not shown) for operable coupling with medical device 206.

Training system 300 further comprises tank 306, image guidance engine 308, signal generator 310, motor controller 312, DBS lead 314, and electrode 316.

Tank 306 can comprise a saline tank configured to simulate brain conductivity.

Image guidance engine 308 is configured for image-guided correction for DBS lead curvature.

Signal generator 310 can comprise a programmable electrical signal generator.

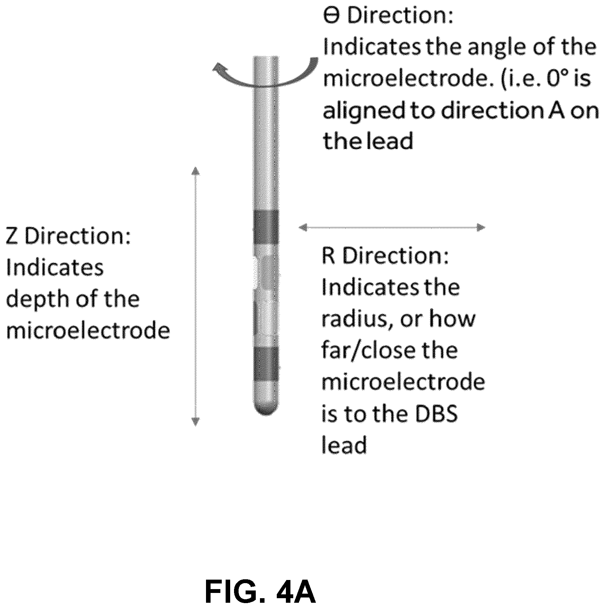

Motor controller 312 can comprise controls for DBS lead rotation (Θ), micro-electrode level (z), and micro-electrode distance (r). For example, referring to FIG. 4A, an annotated diagram of an electrode and the cardinal directions of the electrode (e.g. electrode 316) are depicted, according to an embodiment. DBS lead rotation (Θ) indicates an angle of the microelectrode. Micro-electrode level (z) indicates a depth of the micro-electrode. Micro-electrode distance (r) indicates a radius, or how far or close the micro-electrode is to the DBS lead. Accordingly, the micro-electrode can be configured to physically move. This allows for positioning of the source basically anywhere in relation to the lead. In embodiments, DBS lead 314 is not moved.

DBS lead 314 can comprise an electrical component configured to sense electrical signals, such as simulated brain signals. In an embodiment, DBS lead 314 can be substantially similar to leads 110 of IMD 106.

Electrode 316 can comprise an electrode configured to deliver electrical stimulation according to a target signal. In an embodiment, electrode 316 can be substantially similar to electrode 112 of IMD 106.

In embodiments, training system 300 can further comprise components for vibration suppression and recording stability, such as a limestone table (not shown).

In another embodiment, a training system operates as a 3D electric field tester configured to measure the electrical activity in a saline solution environment. The training system includes a stepper motor configured to physically move the microelectrode, a camera configured to assist in image guided 3D correction for DBS lead curvature, a saline tank to simulate the brain conductivity environment, a limestone table to configured help suppress vibrations and improve recording quality, a DBS lead configured to sense, a microelectrode configured to input a beta signal, and a programmable signal generator configured to control an input signal to the microelectrode.

In embodiments, a training system such as training system 300, or the immediately-described training system embodiment, and, for example, including networked computing device 202) can be operably coupled to a medical device (e.g. medical device 206 or IMD 106) in real-time for programming the device. For example, in an embodiment, one or more in-vivo measurements from medical device 206 can be applied to one or more active simulations on training system 300 such that the one or more active simulations effectively mirror the current conditions of the patient. Based on the results of the simulation, the machine learning algorithms applied, and the visualization(s) output, medical device 206 can be programmed.

In another example, training system 300 can be operably coupled to medical device 206 asynchronously relative to active simulations for programming the device. For example, training system 300 can contain a database of previous simulations (for example, in memory 304), wherein one or more simulations can be selected such that a representation of the patient is obtained. Based on the results of the selected simulation (e.g. based on current conditions of the patient), the machine learning algorithms applied, and the visualization(s) output, medical device 206 can be programmed.

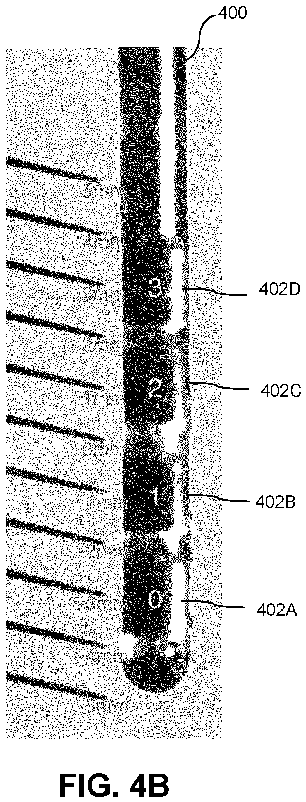

Accordingly, in embodiments, training system 300 is configured to conduct a brain sense survey. For example, referring to FIG. 4B, a diagram of an example lead utilized in training system 300 of FIG. 3 is depicted, according to an embodiment. For ease of explanation, lead 400 is renumbered in FIG. 4B but can correspond to DBS lead 314. And, as will be readily appreciated, lead 400 can likewise be utilized in IMD 106, such as in lead 110. Lead 400 can comprise segmented and/or ring electrodes, such as those depicted in portions 402A-402D.

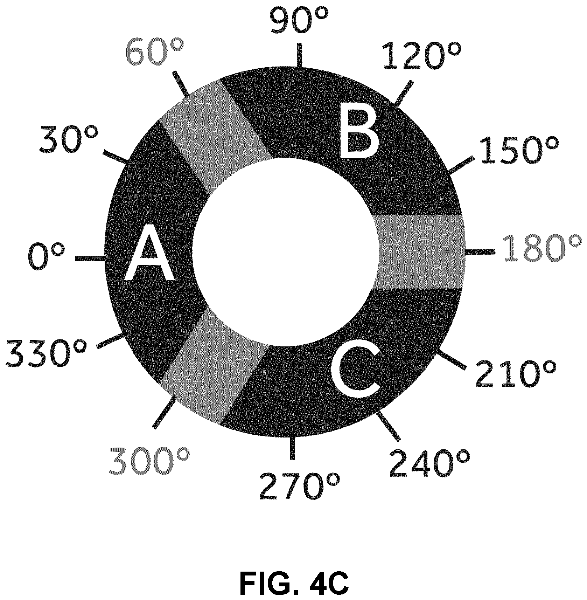

Further, referring to FIG. 4C, an annotated illustration of a cross-section of the lead of FIG. 4B is depicted, according to an embodiment. FIG. 4C depicts theta positions of 0°, 30°, 60°, 90°, 120°, 150°, 180°, 210°, 240°, 270°, 300°, and 330°. Referring also to FIG. 4B, possible micro-electrode distance positions include: 1mm, 1.25mm, 1.5mm, 1.75mm, 2mm, 2.25mm, 2.5mm, 2.75mm, 3mm, 3.25mm, 3.5mm, 3.75mm, 4mm, 4.5mm, 5mm, 6mm, 7mm, 8mm, and possible micro-electrode level positions include: -5mm, -4mm, - 3mm, -2mm, -1mm, 0mm, 1mm, 2mm, 3mm, 4mm, 5mm, resulting in 2376 possible positions, in this example.

Accordingly, a brain sense survey conducted by training system 300 can sense data from every channel that is available on a DBS lead. In embodiments, the local field potential (LFP) is gathered from all possible positions. This is also referred to as sensing montage data. In other embodiments, a brain sense survey can sense data from selected positions instead of all positions.

In an embodiment, a full montage can be taken in multiple steps. For example, based on limitations of channel recording (e.g. a limited number of channels at a time), once data from all relevant sensing pairs is taken, the algorithm can be informed of the corresponding full montage data. Additionally, the brain sense survey can be conducted using bipolar or monopolar configuration.

Referring again to training system 300, using a brain sense survey, a tank simulation set up can be utilized to create machine learning algorithms. A tank simulation set up advantageously allows for the generation of data very easily, and without the patient fatigue of in-vivo models.

Embodiments of the tank simulation determine an oscillatory source as a point source. Contrary to known solutions in which modeling experts asserted that a point source would not adequately represent electrical distribution potential in the brain, the inventors have discovered that the embodiments described herein effectively predict therapy settings for use with an IMD.

Moreover, embodiments of the tank simulation are able to determine the ground truth of the source location. For example, the oscillatory source can be determined according to an x-y-z coordinate location in a tank simulation. Such ground truth locating cannot be done in-vivo or in models created solely with patient pool data.

In certain embodiments, tank simulations can be used for integration of many different devices other than DBS. For example, embodiments can be utilized in any device that is capable of sensing and where the therapy decision making involves localization of an electrode/source. In another advantage, training from tank data does not involve any bias from patient pool sources (such as having patients in the pool that only consist of one phenotype).

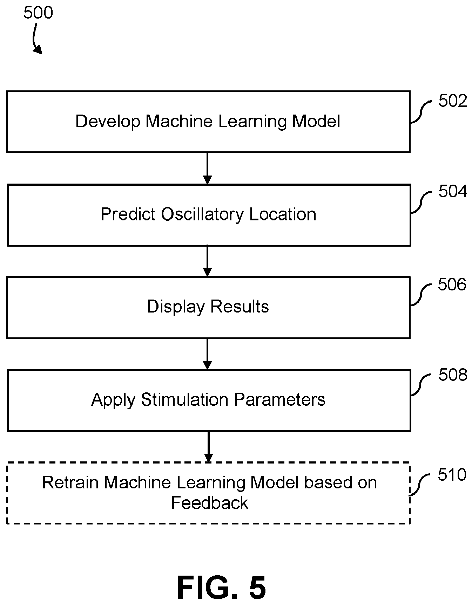

Referring to FIG. 5, a flowchart of a method 500 for programming a medical device is depicted, according to an embodiment. In the following description of FIG. 5, reference is further made to FIGS. 1 and 3.

Method 500 generally comprises developing a machine learning model at 502. In an embodiment, one or more models can be built using bench data, such as that collected from training system 300.

In another embodiment, one or more models can be built using data from a patient data pool. For example, embodiments can utilize a database of multiple patient data. Models can be generated that for example, discriminate by phenotypes. The more alike certain patient data is, the easier algorithms are able to predict. In still other embodiments, individual per-patient models can be built using patient-specific data. In still other embodiments, one or more models can be built using other in-vivo models.

At 502, developing the machine learning model can further include optimizing the machine learning model based on training set data. For example, machine learning algorithms can be utilized for feature selection. Accordingly, machine learning algorithms can be programmed to allow the algorithms to learn various patterns and structures. In certain embodiments, features can be generated from the training set based on prior knowledge. For example, features can be generated based on frequency content.

In embodiments, certain features can be utilized for an oscillatory signal, such as time or frequency. In another example, features can be expand to LFP beta. In an example of a patient with essential tremor, embodiments can utilize frequency across the board (e.g. 5 Hz windows, or other specific frequencies or frequency windows).

Method 500 further comprises predicting one or more oscillatory locations at 504. For example, the optimized machine learning model can be applied to an existing patient's data (e.g. retrieved via IMD 106). In embodiments, an electrode location or mid-commissural point (MCP) coordinates can be reported.

In embodiments, one or more optimal electrodes can be predicted. In DBS programming, stimulation targets can be relatively small (e.g. compared to neurostimulation in other parts of the body), and further, can be proximate regions where stimulation may cause side effects. Accordingly, determining an optimal location is important. As used herein, "optimal" can include an electrode location and/or direction where symptom reduction is highest and/or which avoids side effect regions.

In one example, reducing LFP power leads to better patient outcomes. Accordingly, optimal locating utilizes a target region for symptom reduction as a reduction in LFP beta power. In embodiments, optimal locating can include targeting the largest therapeutic window where stimulation can be applied with room to increase without hitting a side effect region. More particularly, using sensing data, regions of high LFP power can be determined. The regions of high LFP power can then be selected as a target (where a reduction in LFP power would be beneficial).

Regarding whether a therapy setting is beneficial, certain patient conditions can be treated more quickly than others. For example, in treating certain movement disorders such as Parkinson's disease, improvement can be shown in the patient in minutes (compared to months for other patient conditions like epilepsy.) In embodiments, patient response to an initial therapy program or therapy program parameter (such as electrode selection) can be fed back into machine learning algorithms for future predictions.

Method 500 further comprises displaying the results of the prediction of 504 at 506. In an embodiment, oscillatory source location can be visualized with anatomy depicted or without anatomy depicted. In an embodiment, heatmaps can be utilized to indicate one or more optimal electrodes.

Method 500 further comprises applying the simulation parameters to a medical device at 508. In an embodiment, the model prediction at 504 can inform stimulation parameters. For example, a patient medical device can be auto-populated with settings corresponding to the predicted optimal electrode(s). In another example, the predicted optimal electrode(s) can be output as a digital or analog list for subsequent input to the medical device by the clinician (thus obtaining a further safety check implementing human-in-the-loop). Accordingly, clinicians can use the predicted optimal electrode(s) to inform stimulation parameter selection.

Method 500 further optionally comprises retraining the machine learning model based on the feedback of the application of the simulation parameters to the medical device at 510. For example, if a clinician chose a certain predicted electrode to program in medical device 106, the machine learning model can incorporate such affirmative feedback. In another example, if a clinician did not choose a predicted electrode, the machine learning model can incorporate such negative feedback.

In an embodiment, various machine learning algorithms can be utilized. In one embodiment, four machine learning algorithms are utilized in combination for location prediction: closest electrode, radius, depth, and theta. In these embodiments, as described further herein, LFP beta power is a primary parameter, although other parameters are, of course, considered. Embodiments of machine learning algorithms can further utilize multiple frequency features.

For example, in an embodiment to predict stimulation parameters, machine learning algorithms can be applied to a preclinical dataset. Machine learning algorithms can be applied to sensing montage data for each brain target. A predicted electrode can accordingly be generated by: selecting the electrode with the majority of predictions using the closest electrode algorithm and selecting the electrode that aligned with the majority of prediction by combining the depth algorithm and theta algorithm. In another embodiment, the radius, depth, and theta algorithms can be utilized in combination. In an embodiment, predictions can be compared to the electrode that provided the greatest amount of suppression during one or more stimulation experiments, if applicable.

Embodiments described herein can be utilized for initial programming of a medical device. In further embodiments, subsequent or on-going programming can further be conducted. For example, after an initial brain sense survey, a subsequent brain sense survey can be utilized to further program the medical device. The subsequent brain sense survey can integrate specific patient data to reflect disease progression or other states after initial programming.

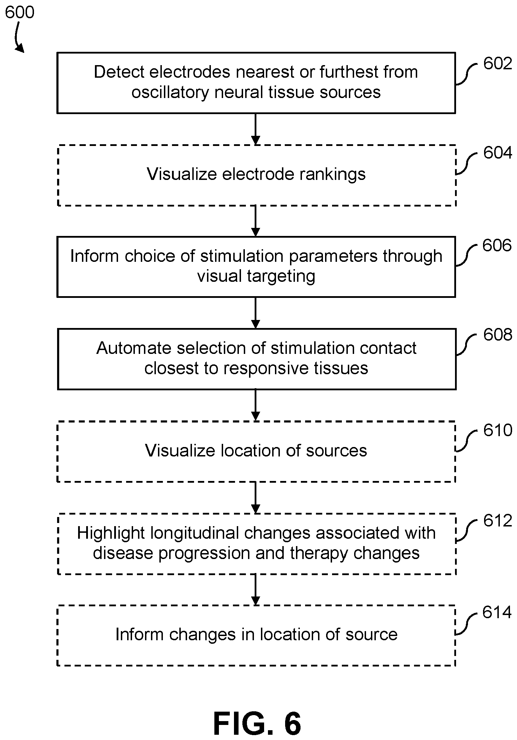

Referring to FIG. 6, a flowchart of another method 600 for programming a medical device is depicted, according to an embodiment.

Method 600 generally comprises detecting one or more electrodes nearest or furthest from an oscillatory neural tissue source at 602. For example, using a brain sense survey in a simulated environment, embodiments can gather montage data from all possible channels of a DBS lead. Subsequently, machine learning algorithm predictions can inform stimulation electrode detection.

At 604, electrode rankings can optionally be visualized based on the electrodes detected at 602. In an embodiment, electrode rankings can be visualized with anatomical scan data ("with anatomy"). In another embodiment, electrode rankings can be visualized without anatomical scan data ("without anatomy").

At 606, a predicted choice of stimulation parameters can be presented through visual targeting. For example, embodiments allow for the viewing of a physiological signal easily within the physical context of anatomy.

At 608, selection of one or more stimulation contacts closest to responsive tissues can be programmed. In certain embodiments, such selection is automated by auto-populating data into a medical device or a programmer for the medical device.

In embodiments, and further to automating selection of stimulation contacts, method 600 further optionally comprises visualizing the location of source(s) with or without anatomical imaging scan data at 610.

In embodiments, and further to automating selection of stimulation contacts, method 600 further optionally comprises highlighting longitudinal changes associated with disease progression and therapy changes at 612.

In embodiments, and further to automating selection of stimulation contacts, method 600 further optionally comprises informing changes in a location of an oscillatory source that may indicate changes in implant location, physiological changes, or other clinically-relevant variables at 614.

For example, in one embodiment of multiple LFP sources, there may be certain oscillatory sources to avoid. Stimulation of certain circuits may result in unwanted side effects or otherwise disrupt beneficial natural physiological dynamics. Embodiments can therefore be used to identify locations to avoid and can be combined with algorithms and/or models that estimate the extent of activation from stimulation to assist in avoidance of stimulation of those locations.

Embodiments of a machine learning algorithm can be used in combination with stimulation to look for changes in source location that result from stimulation. For example, if there are multiple oscillatory sources, embodiments of machine learning algorithms can be utilized to test if stimulation in one direction disrupts the strength of signal on one or more other electrodes (on the same or different lead) and not the other(s).

In another example, embodiments of machine learning algorithms can be utilized check if suppressing the source causes a new source to pop up. In certain embodiments, when a dominant resonance frequency is suppressed, other frequencies often pop up over time (e.g. suppression of one frequency allows the chance for other frequencies to emerge). Sensing the source at different time periods post-op can allow insight into emergence and can allow for adjustment accordingly.

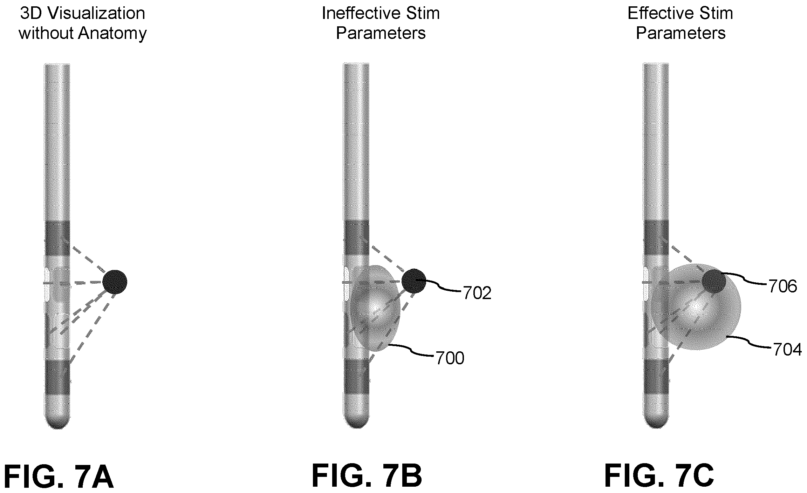

Various visualizations are considered to indicate optimal (or in certain cases for comparison, ineffective or sub-optimal) electrodes. Referring to FIGS. 7A-7C, example location prediction visualizations for a lead are depicted, according to embodiments.

In particular, FIG. 7A generally illustrates a three-dimensional visualization of a location prediction without any anatomy depiction. FIG. 7B illustrates a three-dimensional visualization of a location prediction for ineffective stimulation parameters, again without anatomy. As illustrated, a VNA 704 is depicted proximate the lead relative to an oscillatory source 706. FIG. 7C illustrates a three-dimensional visualization of a location prediction for effective stimulation parameters, again without anatomy. As illustrated, a volume of neural activation (VNA) 700 is depicted proximate the lead relative to an oscillatory source 702. For comparison, in FIG. 7B the VNA 700 is relatively less than the VNA 704 in FIG. 7C indicating ineffective compared to effective parameters.

Accordingly, an oscillatory source can be visualized without the need to depict anatomy, such as with a dot or sphere relative to the lead. Embodiments can therefore integrate with VNA programming settings. In certain embodiments where anatomy is depicted, the source depiction can aid in verifying if the source is the STN.

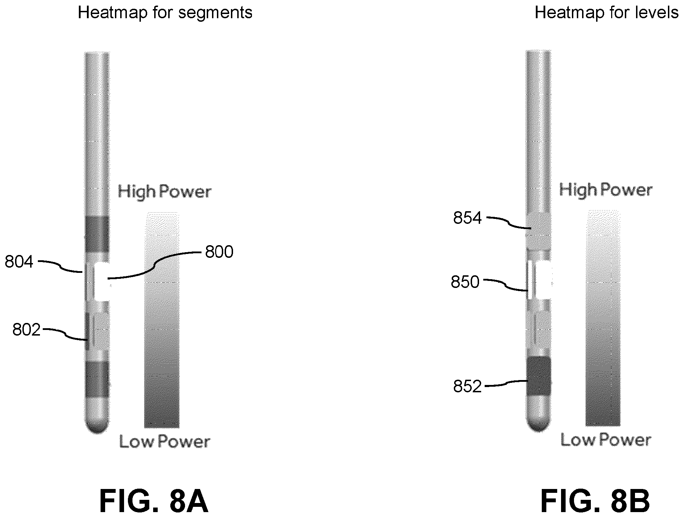

In another example visualization, referring to FIGS. 8A-8B, three-dimensional electrode prediction visualizations for a lead are depicted, according to embodiments. For example, FIG. 8A illustrates a heatmap for various lead segments ranging from high power to low power. Electrode 800 (high power segment) is contrasted with electrode 802 (low power segment) and electrode 804 (relatively medium power segment). In another example, FIG. 8B illustrates a heatmap for various lead segments ranging from high power to low power. Electrode 850 (high power level) is contrasted with electrode 852 (low power segment) and electrode 854 (relatively medium power segment). Accordingly, relative rankings of electrodes can be expressed without the need to depict anatomy. Such rankings can be depicted on the lead itself for ease of clinician application.

In another embodiment, visualization results can be layered or combined with other data types for visualization or confirmation of predictions or to demonstrate overlap with surgical planning.

In one example, other data types can include intra-operative micro-electrode recordings and/or macro-recordings.

In another example, information from a machine learning algorithm can be combined with imaging data to balance and/or weigh the imaging with the electrophysiology. In one particular embodiment, imaging can be utilized to confirm or correlate with the electrode highlighted by machine learning or the location predicted by machine learning (combine depth, theta, and radius) as closest to a source.

In another embodiment, feedback can be added on what to avoid even if the electrode/location highlighted by the machine learning is suggesting or predicting stimulation on certain contacts. For example, imaging can indicate that stimulation in the predicted direction or location may result in side effects. Specifically, if machine learning predicts using a particular electrode/location and the electrode/location is not in place to stimulate as being outside a region of interest or inside a target known to cause side-effects, a message or alert can be provided to physician (not to stimulate there) as a safety feature.

In another embodiment, anatomy can be used to constrain the reporting of electrodes/locations to only those within a particular region of interest.

Embodiments described herein can be incorporated into a reliability measure to help the physician or (automated) system decide whether to use (or even display) the machine learning-based electrode recommendation. For example, if the best electrode location is predicted to be outside of an anatomical structure of interest, the system may not show the physician the intensity for that location (and normalize across the remaining contacts), may provide a warning, or the physician may be able to choose whether to see or not to see machine learning-based values in/out of a region of interest.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

The following examples are useful to understand the invention:
1. A method for programming a medical device, comprising:
   conducting a brain sense survey with at least one lead including a plurality of electrodes in a simulated environment;
   developing at least one machine learning model based on the brain sense survey;
   applying the at least one machine learning model to in-vivo patient data to determine at least one predicted electrode from the plurality of electrodes relative to an oscillatory source;
   visualizing the at least one predicted electrode; and
   programming the medical device based on the at least one predicted electrode.
2. The method of example 1, wherein conducting the brain sense survey includes collecting data from all possible channels for the plurality of electrodes.
3. The method of example 1, wherein visualizing the at least one predicted electrode includes displaying the at least one lead and the at least one predicted electrode proximate anatomical scan data.
4. The method of example 1, wherein visualizing the at least one predicted electrode includes displaying the at least one lead and the at least one predicted electrode without anatomical scan data.
5. The method of example 1, wherein visualizing the at least one predicted electrode includes displaying the at least one lead and the at least one predicted electrode relative to a heatmap.
6. The method of example 1, wherein applying the at least one machine learning model to in-vivo patient data to determine the at least one predicted electrode includes detecting an electrode furthest from the oscillatory source.
7. The method of example 1, wherein applying the at least one machine learning model to in-vivo patient data to determine the at least one predicted electrode includes detecting an electrode nearest to the oscillatory source.
8. The method of example 1, wherein visualizing the at least one predicted electrode includes displaying at least one longitudinal change associated with disease progression or a therapy change.
9. The method of example 1, wherein visualizing the at least one predicted electrode includes displaying a change in a location of the oscillatory source.
10. The method of example 1, wherein the simulated environment includes a signal generated using a signal generator in a saline tank.
11. A system comprising:
   a simulated environment with at least one lead having a plurality of electrodes;
   computing hardware of at least one processor and a memory operably coupled to the at least one processor; and
   instructions that, when executed on the computing hardware, cause the computing hardware to implement: a training sub-system configured to: conduct a brain sense survey using the simulated environment, develop at least one machine learning model based on the brain sense survey, apply the at least one machine learning model to in-vivo patient data to determine at least one predicted electrode from the plurality of electrodes relative to an oscillatory source, visualize the at least one predicted electrode, and program a medical device based on the at least one predicted electrode.
12. The system of example 11, wherein the training sub-system is configured to conduct the brain sense survey by collecting data from all possible channels for the plurality of electrodes.
13. The system of example 11, wherein the training sub-system is configured to visualize the at least one predicted electrode including by displaying the at least one lead and the at least one predicted electrode proximate anatomical scan data.
14. The system of example 11, wherein the training sub-system is configured to visualize the at least one predicted electrode including by displaying the at least one lead and the at least one predicted electrode without anatomical scan data.
15. The system of example 11, wherein the training sub-system is configured to visualize the at least one predicted electrode including by displaying the at least one lead and the at least one predicted electrode relative to a heatmap.
16. The system of example 11, wherein the training sub-system is configured to visualize apply the at least one machine learning model to in-vivo patient data to determine the at least one predicted electrode including by detecting an electrode furthest from the oscillatory source.
17. The system of example 11, wherein the training sub-system is configured to visualize apply the at least one machine learning model to in-vivo patient data to determine the at least one predicted electrode including by detecting an electrode nearest to the oscillatory source.
18. The system of example 11, wherein the training sub-system is configured to visualize the at least one predicted electrode including by displaying at least one longitudinal change associated with disease progression or a therapy change.
19. The system of example 11, wherein the training sub-system is configured to visualize the at least one predicted electrode including by displaying a change in a location of the oscillatory source.
20. The system of example 11, further comprising: a saline tank; and a signal generator configured to generate an electrical signal corresponding to simulated brain activity in the saline tank.

## Claims

1. A method for programming a medical device, comprising:
conducting a brain sense survey with at least one lead including a plurality of electrodes in a simulated environment;
developing at least one machine learning model based on the brain sense survey;
applying the at least one machine learning model to in-vivo patient data to determine at least one predicted electrode from the plurality of electrodes relative to an oscillatory source;
visualizing the at least one predicted electrode; and
programming the medical device based on the at least one predicted electrode.

2. The method of claim 1, wherein conducting the brain sense survey includes collecting data from all possible channels for the plurality of electrodes.

3. The method of any of the preceding claims, wherein visualizing the at least one predicted electrode includes displaying the at least one lead and the at least one predicted electrode proximate anatomical scan data.

4. The method of any of the preceding claims, wherein visualizing the at least one predicted electrode includes displaying the at least one lead and the at least one predicted electrode without anatomical scan data.

5. The method of any of the preceding claims , wherein visualizing the at least one predicted electrode includes displaying the at least one lead and the at least one predicted electrode relative to a heatmap.

6. The method of any of the preceding claims, wherein applying the at least one machine learning model to in-vivo patient data to determine the at least one predicted electrode includes detecting an electrode furthest from the oscillatory source and/or detecting an electrode nearest to the oscillatory source.

7. The method of any of the preceding claims, wherein visualizing the at least one predicted electrode includes displaying at least one longitudinal change associated with disease progression or a therapy change and/or displaying a change in a location of the oscillatory source.

8. The method of any of the preceding claims, wherein the simulated environment includes a signal generated using a signal generator in a saline tank.

9. A system comprising:
a simulated environment with at least one lead having a plurality of electrodes;
computing hardware of at least one processor and a memory operably coupled to the at least one processor; and
instructions that, when executed on the computing hardware, cause the computing hardware to implement: a training sub-system configured to: conduct a brain sense survey using the simulated environment, develop at least one machine learning model based on the brain sense survey, apply the at least one machine learning model to in-vivo patient data to determine at least one predicted electrode from the plurality of electrodes relative to an oscillatory source, visualize the at least one predicted electrode, and program a medical device based on the at least one predicted electrode.

10. The system of claim 9, wherein the training sub-system is configured to conduct the brain sense survey by collecting data from all possible channels for the plurality of electrodes.

11. The system of any of the claims 9 or 10, wherein the training sub-system is configured to visualize the at least one predicted electrode including by displaying the at least one lead and the at least one predicted electrode proximate anatomical scan data.

12. The system of any of the claims 9 to 11, wherein the training sub-system is configured to visualize the at least one predicted electrode including by displaying the at least one lead and the at least one predicted electrode without anatomical scan data.

13. The system of any of the claims 9 to 12, wherein the training sub-system is configured to visualize the at least one predicted electrode including by displaying the at least one lead and the at least one predicted electrode relative to a heatmap.

14. The system of any of the claims 9 to 13, wherein the training sub-system is configured to apply the at least one machine learning model to in-vivo patient data to determine the at least one predicted electrode including by detecting an electrode furthest from the oscillatory source.

15. The system of any of the claims 9 to 14, wherein the training sub-system is configured to apply the at least one machine learning model to in-vivo patient data to determine the at least one predicted electrode including by detecting an electrode nearest to the oscillatory source.
